# EUROPEAN PATENT SPECIFICATION

(11) **EP 4 154 273 B1**
(45) Date of publication and mention of the grant of the patent: **01.07.2026**
(21) Application number: 21732118.1
(22) Date of filing: 20.05.2021
(51) Int. Cl.: G16H 50/50

(54) **METHOD FOR THE SIMULATION OF CORONARY CHANGES AND/OR FOR THE RISK ASSESSMENT OF MYOCARDIAL ISCHEMIA**
VERFAHREN ZUR SIMULATION VON KORONAREN VERÄNDERUNGEN UND/ODER ZUR RISIKOBEWERTUNG VON MYOKARDIALER ISCHÄMIE
PROCÉDÉ DE SIMULATION DE CHANGEMENTS CORONAIRES ET/OU D'ÉVALUATION DU RISQUE D'ISCHÉMIE MYOCARDIQUE

(30) Priority: 22.05.2020 IT 202000012031
(43) Date of publication of application: 29.03.2023
(73) Proprietor: Università Degli Studi di Pavia, 27100 Pavia (PV) (IT); Policlinico San Donato S.p.A., 20097 San Donato Milanese (MI) (IT)
(72) Inventor: LO RITO, Mauro, I-20134 MILANO (IT); CONTI, Michele, 97018 Scicli (RG) (IT)
(74) Representative: Bird & Bird Società tra Avvocati S.r.l.
(86) International application number: PCT/IB2021/054361
(87) International publication number: WO 2021/234616

(56) References cited:
- US-A1- 2013 246 034
- PAOLO ANGELINI ET AL: "Anatomic spectrum of left coronary artery anomalies and associated mechanisms of coronary insufficiency", CATHETERIZATION AND CARDIOVASCULAR INTERVENTIONS, WILEY-LISS, NEW YORK, NY, US, vol. 92, no. 2, 26 July 2018 (2018-07-26), pages 313 - 321, XP072267620, ISSN: 1522-1946, DOI: 10.1002/CCD.27656
- NØRGAARD B L ET AL: "Fractional flow reserve derived from coronary CT angiography in stable coronary disease: a new standard in non-invasive testing?", EUROPEAN RADIOLOGY, SPRINGER INTERNATIONAL, BERLIN, DE, vol. 25, no. 8, 14 February 2015 (2015-02-14), pages 2282 - 2290, XP035508562, ISSN: 0938-7994, [retrieved on 20150214], DOI: 10.1007/S00330-015-3619-1
- KIM SONG SOO ET AL: "Sudden cardiac death from structural heart diseases in adults: imaging findings with cardiovascular computed tomography and magnetic resonance", INTERNATIONAL JOURNAL OF CARDIOVASCULAR IMAGING, KLUWER ACADEMIC PUBLISHERS, DORDRECHT, NL, vol. 32, no. 1, 2 May 2016 (2016-05-02), pages 21 - 43, XP035993276, ISSN: 1569-5794, [retrieved on 20160502], DOI: 10.1007/S10554-016-0891-3
- CRISTINA BASSO ET AL: "Clinical profile of congenital coronary artery anomalies with origin from the wrong aortic sinus leading to sudden death in young competitive athletes", JOURNAL OF THE AMERICAN COLLEGE OF CARDIOLOGY, ELSEVIER, NEW YORK, NY, vol. 35, no. 6, 7 January 2000 (2000-01-07), pages 1493 - 1501, XP028142514, ISSN: 0735-1097, [retrieved on 20110216], DOI: 10.1016/S0735-1097(00)00566-0
- LO RITO MAURO ET AL: "Anomalous aortic origin of coronary artery biomechanical modeling: Toward clinical application", THE JOURNAL OF THORACIC AND CARDIOVASCULAR SURGERY, MOSBY-YEAR BOOK, INC., ST. LOUIS, MO, US, vol. 161, no. 1, 24 August 2020 (2020-08-24), pages 191, XP086397473, ISSN: 0022-5223, [retrieved on 20200824], DOI: 10.1016/J.JTCVS.2020.06.150

## Description

The present invention relates to a method for simulating coronary changes and/or for assessing the risk of myocardial ischemia for congenital coronary anomalies, preferably dynamic stress-induced ischemia. In particular, the present invention relates to a method capable of describing the changes in the coronary artery (anomalous and non-anomalous) at rest and/or under stress through a dynamic, patient-specific (non-static) model, leading to the assessment of the risk of myocardial ischemia, preferably dynamic stress-induced ischemia, of the individual patient.

Congenital anomalies of the coronary arteries originating from the aorta *(Anomalous Aortic Origin of a Coronary Artery* - AAOCA) are rare pathologies with a prevalence ranging from 0.03% to 0.3% of the population depending on the different forms. These congenital anomalies of the coronary arteries can be lethal in people who play sports; this risk is not necessarily linked to the practice of sports but can derive from any physiological and/or pathological condition, comparable to intense physical effort. In AAOCAs, myocardial ischemia and the consequent event of sudden death from cardiac arrest and/or ventricular fibrillation are due to a mechanism not yet fully defined, but which can be summarized as a dynamic stress-induced ischemia.

AAOCA is a congenital defect in which one or more of the coronary arteries originate in an ectopic area of the aorta, such as, for example, from the opposite Valsalva sinus. The left and right coronaries can, in addition to the anomalous origin, go through one of the following five different courses: interarterial, prepulmonary, subpulmonary, retroaortic or retrocardiac. Each of these forms is associated with a different risk of myocardial ischemia, the cause of which varies according to the form and type of anomaly.

In most cases, the disease has no symptoms before sudden death which generally occurs unexpectedly in the recovery phase after moderate or intense physical exercise. Given the stochastic nature of ischemic events and the impossibility of reproducing them in the clinical context through the provocative tests currently available, it is difficult to determine the exact conditions of physical effort that trigger ischemia.

The international guidelines describe a risk profile based on anatomical parameters and on the presence/absence of previous ischemic events.

The indication for surgery then differs between the anomalous aortic origin of the left coronary artery (AAOLCA) or the right coronary artery (AAORCA).

AAOLCA is the least prevalent form (0.03%) and the diagnosis itself indicates the need for surgical treatment as it is considered as being a form with a high risk of sudden death.

AAORCA is the most frequent form with a prevalence of 0.23% and is generally considered to be low-risk. The indication for surgery or medical observation for this second form is based on: a) the presence of angina pectoris and b) ischemic alteration documented by provocative tests with the presence of high-risk anatomical features, such as intramural segments and the morphology of the slit-like coronary ostium.

Although patients with AAORCA are considered to be at low risk, myocardial ischemia as well as sudden death can occur at any age and are not always related to sports activities. Unfortunately, AAORCA can occasionally be found in asymptomatic subjects where previous stress tests have given negative results demonstrating that this method has a low sensitivity and specificity in this context.

Currently there is no test that is capable of reproducing the conditions of a physical exercise sustained for a long period of time capable of inducing ischemia.

Considering the above limitations, surgical treatment is often proposed nowadays when morphological features are detected that are considered as being at high risk for sudden death, such as the length of a possible intramural segment and the presence of a slit ostium, visualized by contrast computed tomography and intravascular ultrasound.

Depending on the variant found, the surgical reimplantation of the anomalous vessel or the so-called *"unroofing"* are currently possible forms of treatment; both treatments however are recognized as being high-risk interventions and it is questionable whether the risks outweigh the potential benefits of preventing sudden long-term death. Furthermore, these surgical manipulations of the coronary arteries can expose the patient to the development of coronary iatrogenic fibrosis in the long term, thus nullifying the effectiveness of primary surgery.

In an ideal test that evaluates the ischemic risk in congenital coronary anomalies, it should be possible to evaluate the blood supply to the abnormal coronary artery in comparison with the oxygen requirement of the myocardium at rest and in relation to physical activity (intensity and duration) without clearly putting the patient at risk at the time of this evaluation. Currently, a diagnostic method capable of measuring coronary flow under conditions of intense effort is not available, therefore a test of this kind is not available, to date. For this reason, the only possible way of reproducing these conditions in total safety is a patient-specific simulation of the aortic root and coronary artery integrated with the simulation of a blood flow that is capable of imitating characteristics of pressure, flow, and consumption of oxygen comparable to actual status induced by the physical effort.

There are currently methods (e.g., CT-FFR) that are capable of evaluating the coronary flow at rest non-invasively within the context of coronary atherosclerotic disease, which, however, has a completely different ischemic mechanism from AAOCA. In atherosclerotic coronary artery disease, in fact, ischemic/infarct events are due to a reduction in the blood flow within the diseased coronary artery. In atherosclerotic coronary artery disease, the diseased coronary artery presents plaques of a different nature inside the lumen (such as, for example, adipose, fibrotic, calcified). These plaques, depending on the size, cause a reduction in the caliber (stenosis) of the affected coronary artery. Stenosis (fixed) causes a reduction in the coronary flow directly proportional to the degree of stenosis caused by the fibro-calcific plaque. From a biomechanical point of view, the plaque, by narrowing the lumen, causes a reduction in the blood flow, but also a reduction in blood pressure between the upstream and downstream tracts of the stenosis. In atherosclerotic disease, the plaque causes a fixed/constant narrowing of the blood flow and this fixed stenosis characteristic is at the basis of all invasive instrumental evaluations (FFR, IVUS, coronary angiography) and non-invasive (CT-FFR, myocardial scintigraphy, etc.), as the reduction in flow is directly proportional to the severity of the stenosis which can be considered fixed when its evaluation is performed. It is therefore known that the assessment of a reduction in caliber, flow or pressure, for estimating static stenosis is at the basis of current assessments of coronary heart disease severity such as the invasive *fractional flow reserve* (FFR) or through *computed tomography* (CT-FFR).

From a clinical point of view, this plaque stenosis is considered critical when it causes a narrowing of the lumen greater than 60-70%, as it can significantly reduce the flow: as the coronary flow cannot increase (as there is a fixed narrowing) with the gradual increase in oxygen demand during exercise, the onset of ischemic electrocardiographic changes, anginal chest pain up to heart attack, is observed.

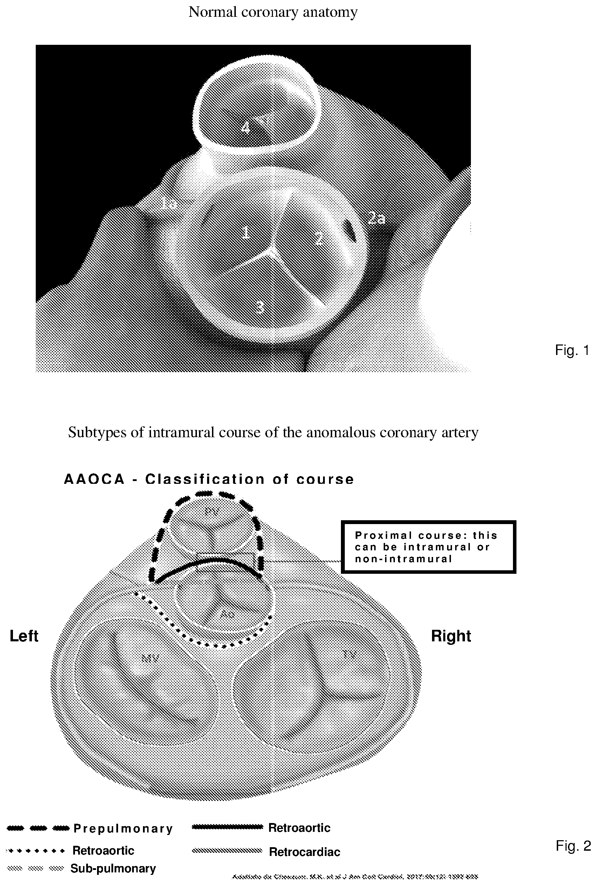

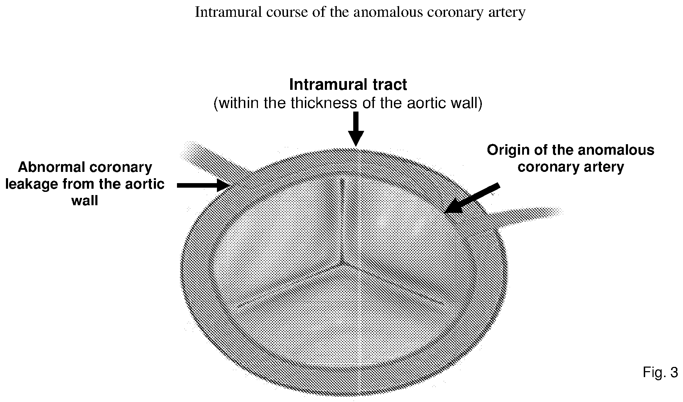

In AAOCA the mechanism is totally different from classic myocardial ischemia due to fixed obstruction by atherosclerotic plaque. Coronaries in AAOCA patients are healthy and have no fixed obstruction plaque. The above-mentioned methods are therefore inadequate for evaluating coronary flow reduction in patients with AAOCA. With respect to the specific anatomical characteristics of this disease, these can be summarized as follows:
1) Origin: the anomalous coronary artery arises from a wrong point of the aorta which, as anticipated above, is generally identified in the Valsalva sinus. Normally, in healthy subjects, the right coronary arises from the right Valsalva sinus, whereas the left coronary arises from the left Valsalva sinus (Figure 1). Coronary arteries that have an abnormal origin can be the left coronary artery (ACL), the right coronary artery (RCA) or other minor branches originating from the wrong Valsalva sinus, ACL from the right or non-coronary Valsalva sinus, and RCA from the left or non-coronary Valsalva sinus.
2) Morphology of the coronary ostium: the anomaly source of the coronary artery is often associated with anomalies of the form of the coronary ostium, which is thus defined as anomalous and which can be (in order of narrowing severity): round> oval> pinhole > slit-like.
3) Course: once the coronary artery originates anomalously, it can take five different paths for reaching its normal distribution territory. These paths can generally be classified as: i) interarterious, ii) subpulmonary, iii) pre-pulmonary, iv) retro-aortic and v) retro-cardiac (Figure 2).
4) Intramural tract: once originating anomalously depending on the course, but especially in the interarterial forms, the initial tract of the anomalous coronary artery can have an intramural course (Figure 3). Intramural course means that the abnormal coronary artery, instead of resting on the external surface of the aortic wall like a normal coronary artery, runs within the thickness of the aortic wall itself. Intramurality determines an oval morphology of the coronary artery and a greater risk of compression and consequent obstruction, which can result in a greater risk of ischemia
*5) Take-off* angle and level: *take-off* angle refers to the angle formed by the origin of the coronary vessel and the aortic wall. In the normal coronary artery, the take-off angle is equal to 90° (coronary artery perpendicular with respect to the aortic wall). In the anomalous coronary artery, the *take-off* angle is generally less than 90° and when it is less than 45° it is defined as acute (defined as high risk). Level refers to the positioning with respect to the normal position (therefore above, or below, the sinotubular junction). If above the junction by more than 5 mm then this is called a *high take-off.*

High-risk anatomical features have been derived from autopsy studies in subjects who died from sudden death or during sports practice. These indications therefore derive from post-mortem evidence and consequently given the nature of this evidence; it is not possible to make a correlation with the functional substrate of the ischemic phenomenon in question.

In any case, the evaluation of the anatomical features, as currently effected, determines a risk profile of the patient, which however has the disadvantage of being obtained through a set of instrumental examinations, as such carried out in basal rest conditions of the patient, which do not allow the behavior of the coronary artery during prolonged effort (sports exercises, or of any other nature) to be evaluated. The assessment of the anatomical features cannot therefore be considered as being well predictive, especially in relation to the effect of prolonged physical effort.

The ischemic mechanism is also extremely difficult to detect and/or provoke even with an exercise test or with any other method that evaluates coronary artery perfusion under stress.

The current study systems of perfusion and/or coronary flow (FFR: *fractional flow reserve*) and simulation of stress tests, in fact, start in any case from the hypothesis and the basic assumption that this is a fixed obstruction to the coronary flow. In this sense, a fixed obstruction is considered easy to simulate and to evaluate.

In the case of anomalous coronary arteries, on the other hand, there is no fixed obstruction (plaque stenosis) inside the vessel, but it is believed that there is a dynamic reduction in caliber or a lack of compensatory dilatation only under stress and therefore, although they may seem similar mechanisms, these are, in fact, very different mechanisms that require a different description and simulation.

AAOCA is a pathology different from coronary atherosclerotic disease and is evidently linked to an important difficulty in life risk stratification, especially considering that the patient is generally also a healthy, asymptomatic subject, who often carries out exercises and/or prolonged physical effort, and in most cases also at a young age. Coronary artery anomalies are reviewed in PAOLO ANGELINI ET AL: "Anatomic spectrum of left coronary artery anomalies and associated mechanisms of coronary insufficiency",CATHETERIZATION AND CARDIOVASCULAR INTERVENTIONS, WILEY-LISS, NEW YORK, NY, US, vol. 92, no. 2, 26 July 2018 (2018-07-26), pages 313-321.

US 2013/246034 covers a method for the assessment of coronary artery stenosis and details a complete workflow that starts from rest-state CT imaging, derives personalised boundary conditions (rest and hyperemic) using clinical data, and computes fractional flow reserve (FFR) with a reduced-order 1-D/semi-empirical model. The need is therefore particularly felt for developing a method for simulating coronary changes, simulating all the stress-induced conditions to which a patient may be subjected, and in particular a patient with an abnormal coronary artery. BRIEF DESCRIPTION of the FIGURES
Figure 1: represents the normal coronary anatomy comprising: 1. Left Valsalva sinus; 1a. Left coronary artery; 2. Right Valsalva sinus; 2a. Right coronary artery; 3. Non-coronary sinus and 4. Pulmonary valve.
Figure 2: represents the possible subtypes of intramural course: i) interarterial, ii) subpulmonary, iii) pre-pulmonary, iv) retro-aortic and v) retrocardiac.
Figure 3: represents the intramural course of an abnormal coronary artery.
Figure 4: schematically represents the *work-flow* of the method, from the acquisition of images and/or *in-vivo* data to the final risk assessment of myocardial ischemia; this work-flow represents the basic scheme of the method of the present invention;
Figure 5: represents the details of the *work-flow,* with identification of the *in-vivo* data and the *in-silico* data of the method.

### DESCRIPTION

The Applicant has found that in a stress situation, the coronary artery, preferably the anomalous coronary artery, reduces its lumen (compression) or fails to sufficiently dilate (lack of adaptation) and this causes a reduction and/or nonincrease in the coronary flow. This condition of coronary blood flow through the coronary artery, in the face of increased demands (such as, for example, in the case of physical effort) causes a mismatch between the supply and demand for oxygen by the myocardium, resulting in ischemic suffering.

This coronary modification takes place under stress in a vessel without a fixed narrowing and is therefore impossible to identify or predict with current predictive or diagnostic methods.

The reduced blood supply in the coronary artery and, in particular, the anomalous coronary artery, may not depend only on coronary alterations under stress, but is a multifactorial functional mechanism determined such as arterial pressure, the extent of stress, cardiac work, and myocardial oxygen demands.

In general, the adverse event (death, arrest or ventricular fibrillation) occurs in a patient after maximum stress or during the first phase of recovery, often in the absence of symptoms or prodromes and not always associated with the intensity of the effort exerted. It is therefore possible that patients have made much greater and more prolonged efforts than those exerted just before the adverse event. This event can also occur after greater efforts, but not necessarily because of the maximum effort reached by the patient.

A new method has now been surprisingly found for the simulation as defined by the claims through a dynamic, patient-specific model (non-static model) of coronary changes (both anomalous and non-anomalous) that take place at rest and under stress, allowing an assessment of the ischemic risk of the individual patient.

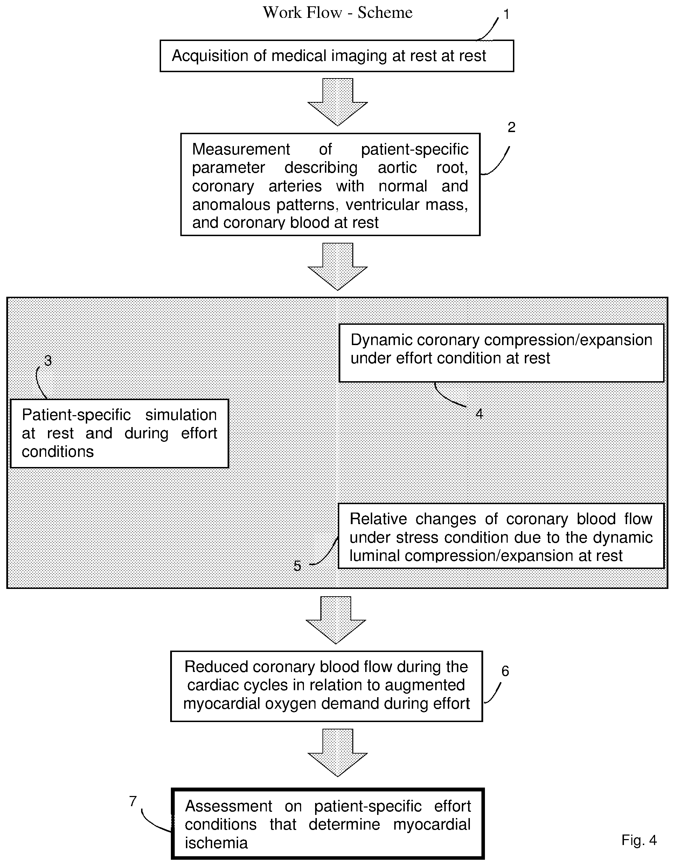

The present invention therefore relates to a method for simulating coronary changes and/or for assessing ischemic risk, preferably dynamic stress-induced ischemia, wherein said method comprises at least the following steps (Figure 4):
a) Acquiring one or more medical images and/or clinical data from the individual patient (1);
b) Extrapolating and/or processing of one or more medical images and/or clinical data of the patient referred to in item a) with the measurement of specific parameters (2);
c) Patient-specific simulation of the aortic root and/or coronary artery at rest and under stress (3) by evaluating the dynamic variations of the coronary dimensions with subsequent compression and/or altered expansion in the stress condition (4);
d) Assessing and quantifying the change in the coronary flow following the stress condition referred to in item c) (5);
e) Assessing the reduced coronary flow in relation to the myocardial requirement during the cardiac cycle at rest and/or stress conditions (6);
f) Determination of the specific stress conditions that can induce myocardial ischemia and consequent assessment of the ischemic risk of the individual patient (7).

According to the present invention, the first step for acquiring one or more medical images and/or clinical data from each individual patient (a) is carried out *in-vivo* and is necessary for obtaining a "specific patient" model, in order to incorporate the specific characteristics of each individual patient under examination, in the method.

The second step (b) is effected through a computerized (*in-silico*) analysis of the images and/or data obtained from the first step (a), which, preferably, according to the invention, describe one or more of the following parameters: i) the aortic root, ii) coronary arteries (both coronary artery with anomaly and coronary artery not affected by anomaly), iii) ventricular mass, iv) coronary flow at rest, v) cardiac output or cardiac flow, vi) arterial pressure and/or vii) heart rate.

The term *in-silico* according to the present invention refers to a mathematical analysis through the computerization of information or data of a chemical, physical or biological nature. This term is therefore used for indicating phenomena of a chemical, physical or biological nature reproduced in a mathematical simulation, rather than in a test-tube or in a living being.

The method according to the present invention is therefore preferably intended as being a method implemented by an electronic processor, i.e., a *computer-implemented method.*

These measures serve for feeding the method of the invention by obtaining the patient-specific simulation of the condition both at rest and under stress.

Simulation of conditions under stress refers to the possibility of virtually evaluating the working conditions of the coronary arteries and myocardium linked to an increased rate of cardiac parameters (cardiac output, pressure, oxygen consumption, etc...).

In this way, according to the method of the invention, the change in the dynamic condition of the coronary arteries and their compression/expansion during stress conditions, which currently cannot be measured and/or estimated in an instrumental and non-invasive way, are simulated.

Using a mathematical model, it is possible to estimate the interaction between aortic and coronary roots (abnormal or not) at different workloads (cardiac output, pressure, oxygen consumption, etc...) and then quantify the changes in the coronary lumen in relation to changes in the aorta under progressively increasing work-load conditions.

With these measurements, the method of the present invention also allows the change in coronary flow under stress conditions to be deduced, due to the expansion and/or compression conditions determined by the simulation previously carried out.

According to the present invention, the images and the data acquired and/or generated are divided into two categories: *in-vivo* data, i.e. measured or obtained on and from the single patient *in-vivo* (as described in point a) of the method of the invention) and data measured or obtained *in-silico,* i.e., data obtained or measured by computer, preferably by computerized mathematics.

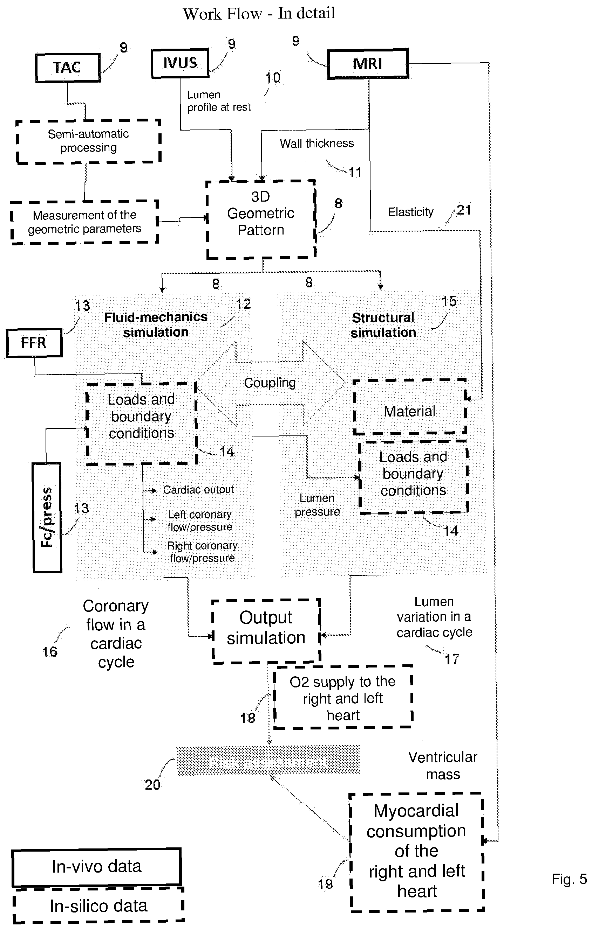

In Figure 5 the data obtained *in-vivo* are highlighted in the flow chart with a continuous frame, whereas the frames in a dashed line define the data obtained or measured *in-silico.*

The *in-vivo* data according to the present invention preferably derive from imaging such as, for example, computerized tomography (CT), intravascular ultrasound (IVUS), magnetic resonance imaging (MRI), heart rate (HR) and/or arterial pressure (press) and intracoronary pressure measurements (Figure 5; 9 and 13).

The *in-silico* data according to the present invention derive from the computerized analysis of the information which is preferably obtained from the *in-vivo* data previously collected.

The hybrid *in-vivo*/*in-silico* combination of the method of the present invention is surprisingly capable of arriving at a risk assessment of the coronary compression (abnormal or non-abnormal coronary artery, preferably abnormal coronary artery) and relative lack and/or reduction in the blood supply during effort, wherein said assessment is patient-specific, personalized, capable of providing a limit of physical activity, beyond which the patient is at risk of ischemia. According to the present invention, myocardial ischemia is preferably myocardial ischemia due to effort.

According to a preferred embodiment of the present invention, as schematized for example in Figure 5, the parameters are extracted from the TAC (9) through a semi-automatic computerized process based on image segmentation and/or analysis with artificial intelligence to create a 3D geometric model of the aortic root and coronaries (8). According to an embodiment of the present invention, the measurements effected on the form of the cross section of the lumen (10) deriving *in vivo* from the IVUS (9) and magnetic resonance imaging (MRI) (9) preferably contribute to the definition of this 3D geometric model (8), from which the thicknesses of the vascular walls of interest (11) are derived.

According to a preferred embodiment of the invention, this 3D model (8) represents a fundamental part of the *in-silico* data and is an important element for linking the data obtained *in-vivo* on the specific patient with the simulation models (12 and 15).

According to a preferred embodiment, this 3D geometric model (8) is therefore the input for the fluid mechanics simulation of the coronary artery (12).

According to the method of the invention, the fluid mechanics simulation (12) can represent both the anomalous coronary artery and the non-anomalous coronary artery, preferably the anomalous coronary artery.

According to an embodiment of the invention, said fluid mechanics simulation of the coronary artery (12) can also be fed directly with patient-specific *in-vivo* data such as, for example, heart rate (HR) and/or arterial pressure (press) (13) to define the boundary and/or working conditions (14) with the relative intervals and/or loads necessary for solving the fluid mechanics analysis.

According to this embodiment of the method according to the present invention, the patient-specific *in-vivo* data contribute, together with the 3D geometric model, to the representation of the fluid-mechanics simulation of the coronary artery of the patient under examination, thus obtaining a patient-specific coronary simulation.

According to an embodiment of the invention, the fluid mechanics simulation of the coronary artery allows the precise blood-pressure distribution to be obtained, which, in turn, allows the enlargement and/or narrowing of the coronary artery to be calculated; as indicated above, the coronary artery can be an anomalous coronary artery or a non-anomalous coronary artery, preferably an anomalous coronary artery.

According to a further embodiment of the invention, the fluid mechanics simulation of the coronary artery (12) allows the solid mechanics simulation (15) to be derived; in accordance with this embodiment of the invention, the measurements carried out on the patient *in vivo* (in a patient-specific way) can be used for defining the mechanical properties of the vascular/cardiac tissue. The measurements obtained *in vivo* with MRI (Magnetic Resonance) and/or TAC (9) preferably contribute to obtaining the solid simulation of the coronary artery (15), together with those deriving from the concomitant fluid mechanics simulation of the coronary artery itself (12). The mechanical properties of the vascular/cardiac tissue according to the method of the invention are, for example, the elasticity, the anisotropy, the compressibility of the biological tissue and/or the dispersion of collagen fibers (21).

Thanks to the method of the present invention, the integration and mutual interaction of the fluid mechanics simulation of the coronary artery (12) with the solid simulation (15) allows the coronary flow during the cardiac cycle (16) and the section variation of the vessel during this cycle (17) to be assessed, both under rest conditions and under stress-induced conditions. With the method of the invention, it also becomes possible to selectively evaluate the oxygen flow-rate for the right and left coronary arteries responsible for the perfusion of the relative cardiac sectors (18).

According to the method of the invention, these data can be compared with the need/consumption of the myocardium which is estimated from the *in-vivo* data on the specific patient (19), preferably through the measurement of the ventricular mass by MRI, obtaining the indication of the risk of ischemia and/or myocardial infarction (20).

As previously observed, this assessment is not currently available through the tests and measurements normally used.

According to an embodiment of the present invention, the method comprises a compression mechanism and/or insufficient dilation due to caliber alterations of the anomalous coronary artery, without obstructive internal plaque, under stress conditions. The mechanism of compression and/or insufficient dilation according to the method of the invention is performed as, under stress conditions, the aorta is subject to dilation due to an increase in the internal pressure; this dilatation alters the caliber of the lumen of the coronary artery and causes a reduction and/or nonincrease in the coronary blood flow.

The compression mechanism and/or insufficient dilatation according to the present invention is without obstructive internal plaque and consequently not related to a scenario of fixed stenosis in the coronary artery. The compression mechanism and/or insufficient dilation according to the present invention determines a dynamic change in the size of the coronary artery, in which the modification of the flow or the lack of vascular increase are not due to pharmacological induction (as, for example, given by the administration of vasodilating agents such as adenosine or papaverine in a stenosis context) but is due to the simulated modification of the physiological parameters typical of physical effort.

The quantification of the coronary flow during effort and/or in the recovery phase for each cardiac cycle in relation to the heart rate allows those critical conditions in which an imbalance is established between the oxygen supply and demand of the heart muscle responsible for ischemia, to be determined. The myocardial ischemia according to the present invention is preferably a dynamic stress-induced ischemia.

An advantage of the method according to the present invention is the synergy expressed by the *in-vivo*/*in-silico* model which is capable of dynamically and nonstatically assessing coronary compression and/or non-expansion and the relative lack of blood supply during effort.

A further advantage of the method of the present invention lies in its parametric nature, with the versatility of the computational approach that allows the images and data upstream of the method to be easily managed. In the method of the present invention, thanks to the interconnection of *in-vivo*/*in-silico* data, it is possible to modify or intervene on a single parameter at a time (for example, a morphological parameter such as intramural length or a physiological parameter such as aortic pressure or a different parameter such as the duration of stress) to simulate all possible risk scenarios for the individual patient. The combination of the images and in-vivo data with the *in-silico* computerized analysis allows the risk of ischemia to be assessed and provides a clinical/physical condition limit for each individual patient, beyond which there is an increased risk of ischemia. Thanks to this method, it is surprisingly possible to obtain an ischemic stratification of the risk and consequently a better indication of the surgical or medical treatment, if necessary, for each individual patient.

Thanks to this method, it will be possible to provide safe intervals of physical effort that will allow the patient, in particular a young patient, to return to normal life and to monitor physical activity without the psychological burden of running the risk of an adverse, sudden, and unpredictable, event at a cardiological level.

A further advantage of the method of the present invention is the possibility of inserting all the functional parameters and being able to simulate a stress test by increasing the cardiac work (cardiac output function, heart rate and blood pressure and oxygen consumption), with the assessment of coronary perfusion in relation to the change in coronary characteristics under stress. In this way, the method of the invention makes it possible to assess the relationship between oxygen supply at the myocardial level and oxygen consumption in relation to the myocardial mass. The method of the present invention thus allows the risk areas or limits of the various parameters linked to the particular ischemic process to be identified in subjects with AAOCA. The aortic and coronary changes are dynamically simulated in a customized model on the individual patient, to identify ischemic risk in a specific way for each patient.

## Claims

1. A computer-implemented method for the simulation of coronary changes and/or the risk assessment of myocardial ischemia, wherein said method comprises the following steps:
a) Acquiring one or more medical images and/or clinical data from each single patient (1);
b) Extrapolating and/or processing one or more medical images and/or clinical data of the patient according to item a) with the measurement of specific parameters (2);
c) Patient-specific simulation of the aortic root and/or coronary arteries at rest and under stress (3) by assessing the dynamic changes in the coronary dimensions with subsequent compression and/or altered expansion under stress conditions (4):
d) Assessing and quantifying the changes in the coronary flow under stress conditions according to item c) (5);
e) Assessing the reduced coronary flow with respect to the myocardial requirement during the cardiac cycle at rest and/or under stress (6);
f) Determining the specific stress conditions that may induce myocardial ischemia and the subsequent risk assessment of ischemia in each single patient (7),
wherein:
• said medical images and/or clinical data are acquired from the single patient, in vivo, at rest and/or under stress;
• wherein said medical images and/or clinical data according to item a) are acquired by means of: computed tomography (TAC), intravascular ultrasounds (IVUS), magnetic resonance imaging (MRI), heart-rate measurements (HR), arterial pressure (press) and/or intracoronary pressure (FFR) and
• wherein said coronary artery is an anomalous coronary artery (AAOCA).

2. The method according to claim 1, wherein said specific parameters stated under item b) describe the aortic root, the coronaries, the ventricular mass, and the coronary flow at rest.

3. The method according to claim 1, wherein said risk assessment of ischemia according to item f) is patient-specific.

4. The method according to any of the previous claims, wherein said myocardial ischemia is dynamic stress-induced ischemia.

## Patentansprüche

1. Computerimplementiertes Verfahren zur Simulation von Koronarveränderungen und/oder zur Risikobewertung einer Myokardischämie, wobei das Verfahren die folgenden Schritte umfasst:
a) Erfassen eines oder mehrerer medizinischer Bilder und/oder klinischer Daten von jedem einzelnen Patienten (I);
b) Extrapolieren und/oder Verarbeiten eines oder mehrerer medizinischer Bilder und/oder klinischer Daten des Patienten gemäß Punkt a) mit der Messung bestimmter Parameter (2);
c) Patientenspezifische Simulation der Aortenwurzel und/oder Koronararterien in Ruhe und unter Belastung (3) durch Bewertung der dynamischen Veränderungen der Koronardimensionen mit anschließender Kompression und/oder veränderter Expansion unter Belastungsbedingungen (4):
d) Bewerten und Quantifizieren der Veränderungen des Koronarflusses unter Belastungsbedingungen gemäß Punkt c) (5);
e) Bewerten des reduzierten Koronarflusses in Bezug auf den myokardialen Bedarf während des Herzzyklus in Ruhe und/oder unter Belastung (6);
f) Bestimmen der spezifischen Belastungsbedingungen, die eine Myokardischämie induzieren können, und die anschließende Risikobewertung der Ischämie bei jedem einzelnen Patienten (7),
wobei:
• die medizinischen Bilder und/oder klinischen Daten von dem einzelnen Patienten in vivo, in Ruhe und/oder unter Belastung erfasst werden;
• wobei die medizinischen Bilder und/oder klinischen Daten gemäß Punkt a) mittels: Computertomographie (TAC), intravaskulärem Ultraschall (IVUS), Magnetresonanztomographie (MRT), Herzfrequenzmessungen (HR), arteriellem Blutdruck (Druck) und/oder intrakoronarem Druck (FFR) erfasst werden und
• wobei die Koronararterie eine anomale Koronararterie (AAOCA) ist.

2. Verfahren nach Anspruch 1, wobei die unter Punkt b) genannten spezifischen Parameter die Aortenwurzel, die Koronare, die ventrikuläre Masse und den Koronarfluss in Ruhe beschreiben.

3. Verfahren nach Anspruch 1, wobei die Risikobewertung der Ischämie gemäß Punkt f) patientenspezifisch ist.

4. Verfahren nach einem der vorhergehenden Ansprüche, wobei die Myokardischämie eine dynamische belastungsinduzierte Ischämie ist.

## Revendications

1. Procédé mis en œuvre par ordinateur pour la simulation de modifications des artères coronaires et/ou l'évaluation du risque d'ischémie myocardique, dans lequel ledit procédé comprend les étapes suivantes :
a) l'acquisition d'une ou plusieurs images médicales et/ou de données cliniques de chaque patient individuel (I) ;
b) l'extrapolation et/ou le traitement d'une ou plusieurs images médicales et/ou de données cliniques du patient conformément au point a) avec la mesure de paramètres spécifiques (2) ;
c) la simulation spécifique au patient de la racine aortique et/ou des artères coronaires au repos et sous stress (3) par l'évaluation des variations dynamiques des dimensions des artères coronaires avec compression et/ou expansion altérée subséquentes sous des conditions de stress (4) :
d) l'évaluation et la quantification des variations du débit coronaire sous des conditions de stress conformément au point c) (5) ;
e) l'évaluation du débit coronaire réduit par rapport aux besoins myocardiques pendant le cycle cardiaque au repos et/ou sous stress (6) ;
f) la détermination des conditions de stress spécifiques susceptibles d'induire une ischémie myocardique et l'évaluation subséquente du risque d'ischémie chez chaque patient individuel (7),
dans lequel :
• lesdites images médicales et/ou données cliniques sont acquises auprès du patient individuel, in vivo, au repos et/ou sous stress ;
• dans lequel lesdites images médicales et/ou données cliniques selon le point a) sont acquises au moyen de : tomodensitométrie (TAC), ultrasons intravasculaires (IVUS), imagerie par résonance magnétique (IRM), mesures de la fréquence cardiaque (HR), pression artérielle (press) et/ou pression intracoronaire (FFR) et
• dans laquelle ladite artère coronaire 1 est une artère coronaire anormale (AAOCA).

2. Procédé selon la revendication 1, dans lequel lesdits paramètres spécifiques mentionnés au point b) décrivent la racine aortique, les artères coronaires, la masse ventriculaire et le débit coronaire au repos.

3. Procédé selon la revendication 1, dans lequel ladite évaluation du risque d'ischémie selon le point f) est spécifique au patient.

4. Procédé selon l'une quelconque des précédentes revendications, dans lequel ladite ischémie myocardique est une ischémie induite par un stress dynamique.
